(19)

Europäisches
Patentamt
European
Patent Office
Office européen
des brevets

(11) **EP 4 209 189 A1**

(12) **EUROPEAN PATENT APPLICATION**
published in accordance with Art. 153(4) EPC

(43) Date of publication:
**12.07.2023 Bulletin 2023/28**

(21) Application number: **21863209.9**

(22) Date of filing: **25.03.2021**

(51) International Patent Classification (IPC):
**A61B 18/12** (2006.01)    **A61B 18/14** (2006.01)
**A61B 5/053** (2021.01)    **A61B 90/00** (2016.01)

(52) Cooperative Patent Classification (CPC):
**A61B 5/053; A61B 18/12; A61B 18/14; A61B 90/00**

(86) International application number:
**PCT/CN2021/083077**

(87) International publication number:
**WO 2022/048138 (10.03.2022 Gazette 2022/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.09.2020   CN 202010918869
03.09.2020   CN 202021909126 U**

(71) Applicant: **Hangzhou Nuo Cheng Medical
Instrument Co., Ltd
Hangzhou, Zhejiang 310000 (CN)**

(72) Inventors:
• **LIU, Daoyang
Hangzhou, Zhejiang 310000 (CN)**
• **WANG, Xiongzhi
Hangzhou, Zhejiang 310000 (CN)**
• **HU, Shanfeng
Hangzhou, Zhejiang 310000 (CN)**
• **QIU, Xinjiong
Hangzhou, Zhejiang 310000 (CN)**

(74) Representative: **Hamer, Christopher K.
Mathys & Squire
The Shard
32 London Bridge Street
London SE1 9SG (GB)**

(54) **LIVING BODY IMPEDANCE DETECTION DEVICE AND RADIO FREQUENCY ABLATION
SYSTEM**

(57)    An impedance detection device (100) for a living body and a radiofrequency ablation frequency (1000) are provided. The detection device (100) includes an excitation-signal generation unit (31), a transmission unit (32), an impedance-signal acquisition unit (33), and a processing unit (35). The excitation-signal generation unit (31) is configured to generate and output a high-frequency excitation signal. The transmission unit (32) is configured to transmit the high-frequency excitation signal to a detection site (21) of the living body via an output port of the transmission unit (32), where the output port of the transmission unit (32) forms an impedance-signal detection point (322). The impedance-signal acquisition unit (33) is electrically connected with the impedance-signal detection point (322), and configured to acquire a sampling signal from the impedance-signal detection point (322) in real time. The processing unit (35) is electrically connected with the impedance-signal acquisition unit (33), and configured to acquire the sampling signal and determine a real-time impedance value of the living body corresponding to a real-time sampling value of the sampling signal according to an impedance calibration data table preset. The impedance detection device (100) for the living body can accurately and quickly detect the real-time impedance value of the living body during treatment, so that the change of the impedance of the living body can be monitored in time during treatment.

FIG. 1

**Description**

CROSS-REFERENCE TO RELATED APPLICATION(S)

This application claims priority to Chinese Patent Application No. 202010918869.X and 202021909126.8, filed to the China National Intellectual Property Administration on September 3, 2020 and entitled "IMPEDANCE DETECTION DEVICE FOR LIVING BODY AND RADIOFREQUENCY ABLATION SYSTEM", the disclosures of which are hereby incorporated by reference in their entireties.

TECHNICAL FIELD

This disclosure relates to the field of impedance detection technology, and particularly to an impedance detection device for a living body and a radiofrequency ablation system.

BACKGROUND

Ablation therapy refers to delivery of energy such as radio frequency, microwave, freezing, and laser to target tissue for ablation under guidance of images, where ablation therapy is minimally invasive and has been applied in clinical treatment of tumor diseases, neurological diseases, and heart diseases such as hypertrophic cardiomyopathy and so on.

An impedance of a living body, such as a human body, is an important electrophysiological signal. During ablation therapy, the impedance of the living body may continuously change with progress of ablation therapy. However, if the impedance of the living body changes too quickly or is too high, there may be a risk of excessive ablation and tissue carbonization in a target area. Therefore, in order to ensure the patient's own safety and the effect of ablation therapy, it needs to detect the impedance of the living body in real time. Some existing solutions for detecting the impedance of the living body are complex and have a slow dynamic response, and thus are not suitable for real-time and rapid detection of the impedance of the living body during ablation therapy.

SUMMARY

The present disclosure provides an impedance detection device for a living body and a radiofrequency ablation system, which can detect an impedance of the living body in real time during treatment, can respond quickly, and have a simple structure, so that the change of the impedance of the living body can be monitored in real time during treatment to ensure safe operation of treatment.

An impedance detection device for a living body is provided in a first aspect of the present disclosure. The impedance detection device for the living body includes an excitation-signal generation unit, a transmission unit, an impedance-signal acquisition unit, and a processing unit. The excitation-signal generation unit is configured to generate and output a high-frequency excitation signal. The transmission unit includes a first transmission port and a second transmission port, where the transmission unit is electrically connected with the excitation-signal generation unit via the first transmission port to receive the high-frequency excitation signal, and is configured to transmit the high-frequency excitation signal to a detection site of the living body via the second transmission port, and the second transmission port forms an impedance-signal detection point. The impedance-signal acquisition unit is electrically connected with the impedance-signal detection point, and configured to acquire a sampling signal from the impedance-signal detection point in real time. The processing unit is electrically connected with the impedance-signal acquisition unit, and configured to acquire the sampling signal and determine a real-time impedance value of the living body corresponding to a real-time sampling value of the sampling signal according to an impedance calibration data table preset, where the impedance calibration data table pre-records a mapping relationship between multiple simulated impedance values of the living body and sampling values of multiple sampling signals.

A radiofrequency ablation system is provided in a second aspect of the present disclosure. The radiofrequency ablation system includes a radiofrequency energy generation unit, an ablation device, and the impedance detection device for the living body mentioned above. The radiofrequency energy generation unit is configured to provide radiofrequency energy for radiofrequency ablation. The ablation device is electrically connected with the radiofrequency energy generation unit, is configured to be inserted into a treatment site of the living body during radiofrequency ablation, to receive the radiofrequency energy output from the radiofrequency energy generation unit, and to release the radiofrequency energy to the treatment site to perform radiofrequency ablation on the treatment site.

The impedance detection device for the living body of the present disclosure determines the real-time impedance value of the living body according to the characteristics that impedance values of the living body and sampling values of sampling signals have an approximately linear relationship within a certain range and the impedance calibration data table preset, and thus the impedance detection device for the living body can respond quickly and can accurately and quickly detect the real-time impedance value of the living body, so that the change of the impedance of the living body can be monitored in real time during treatment, thereby providing reference information for medical staff to ensure safe operation of treatment.

BRIEF DESCRIPTION OF THE DRAWINGS

To describe technical solutions in embodiments of the present disclosure more clearly, the following will briefly introduce the accompanying drawings required for describing the embodiments or related art. Apparently, the accompanying drawings in the following illustration merely illustrate some embodiments of the present disclosure. Those of ordinary skill in the art may also obtain other accompanying drawings based on these accompanying drawings without creative efforts.

FIG. 1 is a schematic structural diagram of an impedance detection device for a living body provided by embodiments of the present disclosure.

FIG. 2 is a schematic diagram of an impedance loop provided by embodiments of the present disclosure.

FIG. 3 is a schematic diagram of circuit structures of an excitation-signal generation unit and a transmission unit in FIG. 1.

FIG. 4 is a schematic diagram illustrating a waveform conversion procedure of the excitation-signal generation unit in FIG. 3.

FIG. 5 is a schematic diagram of a circuit structure of an impedance-signal acquisition unit in FIG. 1.

FIG. 6 is a schematic diagram of a circuit structure of an operational amplification unit in FIG. 1.

FIG. 7 is a schematic diagram of an equivalent circuit of the impedance loop in the present disclosure.

FIG. 8 is a schematic diagram illustrating a curve relationship between voltage values of sampling signals and impedance values of the living body in the present disclosure.

FIG. 9 is a schematic diagram illustrating an impedance calibration data table provided by embodiments of the present disclosure.

FIG. 10 is a flow chart of an impedance detection method for a living body provided by embodiments of the present disclosure.

FIG. 11 is a flow chart of another impedance detection method for a living body provided by embodiments of the present disclosure.

FIG. 12 is a schematic structural diagram of a radiofrequency ablation system provided by embodiments of the present disclosure.

Main reference signs: impedance detection device 100; excitation-signal generation unit 31; first input port 311; first output port 312; waveform conversion circuit 313; transmission unit 32; first transmission port 321; second transmission port (impedance-signal detection point) 322; impedance-signal acquisition unit 33; second input port 331; second output port 332; anti-interference circuit 333; operational amplification unit 34; third input port 341; third output port 342; filter circuit 343; operational amplification circuit 344; processing unit 35; data acquisition module 351; calculation module 352; neural electrode 41; memory 42; capacitors C1, C2, C3, C4, C7, C8, C9, and C10; resistors R1, R2, R3, R5, and R6; inductors L1, L2, and L3; operational amplifiers AR1, AR2, and AR3; living body 200; detection site 21; radiofrequency ablation system 1000; radiofrequency energy generation unit 500; ablation device 600; steps 1001-1002 and 1101-1106.

The present disclosure will be further illustrated in combination with the following specific embodiments and the abovementioned accompanying drawings.

DETAILED DESCRIPTION

The following will clearly and completely describe the technical solutions in the embodiments of the present disclosure with reference to the accompanying drawings in the embodiments of the present disclosure. The accompanying drawings are used for illustrative purposes only, represent only schematic diagrams, and should not be construed as limitations on the present disclosure. Apparently, the described embodiments are only some, not all, embodiments of the present disclosure. Based on the embodiments in the present disclosure, all other embodiments obtained by those of ordinary skill in the art without creative efforts belong to the scope of protection of the present disclosure.

Unless defined otherwise, all technical and scientific terms used in the present disclosure have the same meaning as commonly understood by those of ordinary skill in the art. The terms used in the description of the present disclosure is only for the purpose of describing specific embodiments, and are not intended to limit the present disclosure.

The present disclosure provides an impedance detection device for a living body, which is configured to detect an impedance value of the living body during treatment to monitor the change of the impedance value of the living body, thereby assisting safe operation of treatment. Treatment may include but is not limited to ablation, coagulation, and the like. In the present disclosure, the impedance detection device for the living body is illustrated by taking the application of the impedance detection device for the living body in radiofrequency ablation as an example.

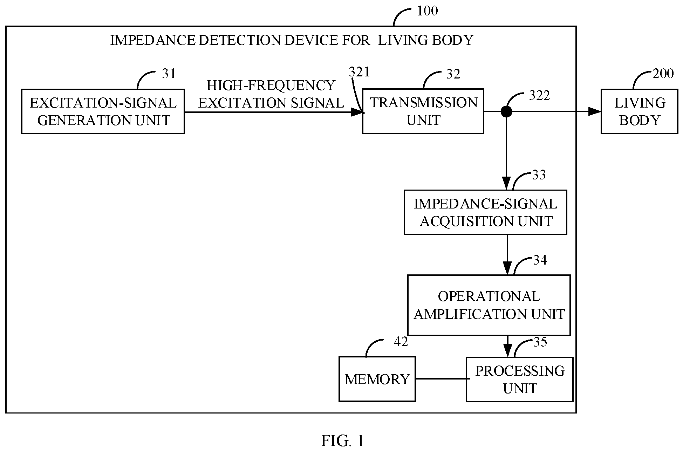

FIG. 1 is a schematic structural diagram of an impedance detection device for a living body provided by embodiments of the present disclosure. As illustrated in FIG. 1, an impedance detection device 100 for a living body 200 includes an excitation-signal generation unit 31, a transmission unit 32, an impedance-signal acquisition unit 33, and a processing unit 35. The excitation-signal generation unit 31 is configured to generate and output a high-frequency excitation signal.

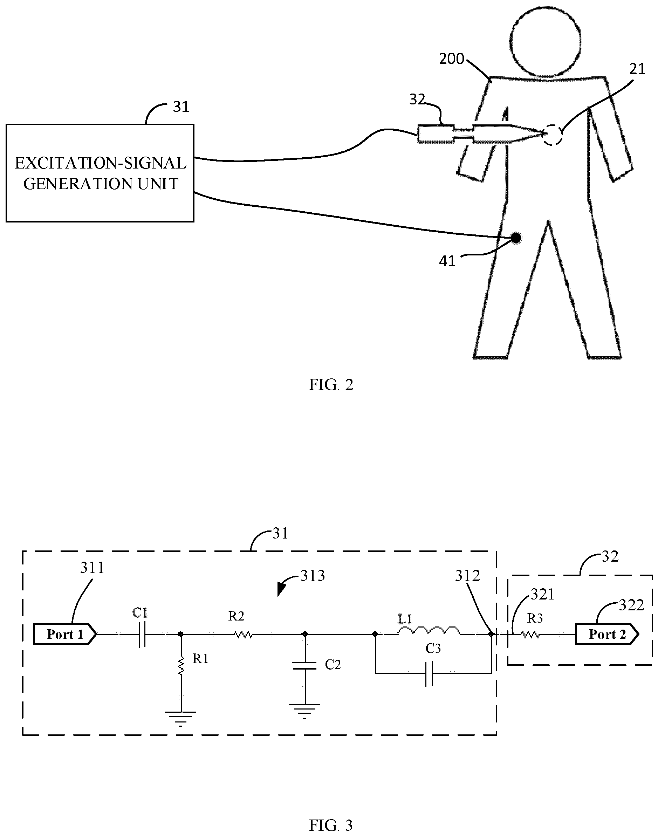

The transmission unit 32 includes a first transmission port 321 and a second transmission port 322. The transmission unit 32 is electrically connected with the excitation-signal generation unit 31 via the first transmission port 321 to receive the high-frequency excitation signal, and is configured to transmit the high-frequency excitation signal to a detection site 21 (as illustrated in FIG. 2) of the living body 200 via the second transmission port 322.

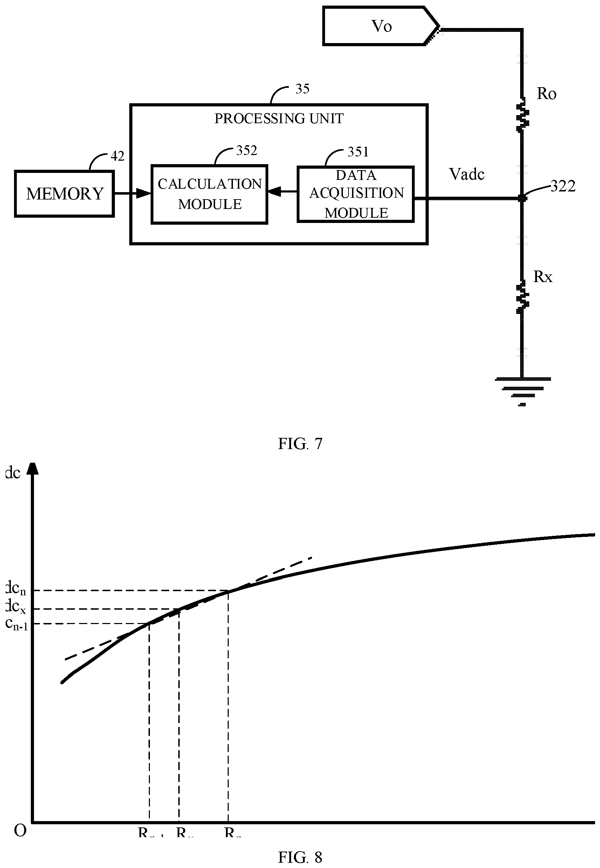

As illustrated in FIG. 2, the living body 200 may be a human body or other animals. In the embodiment, taking the human body as an example, the detection site 21 is a treatment site, and the treatment site may be, for example, the heart. The impedance detection device 100 for the living body 200 may also include a neutral electrode 41 electrically connected with the excitation-signal generation unit 31. The neutral electrode 41 is attached to a suitable position of the patient's body (i.e., the living body 200), such as the leg or the back surface, etc. during treatment. An impedance loop from an output port of the excitation-signal generation unit 31 to the transmission unit 32, the living body 200, and the neutral electrode 41 is formed, so that the high-frequency excitation signal can be transmitted in the impedance loop. An equivalent circuit diagram of the impedance loop is illustrated in FIG. 7. In other embodiments, the detection site 21 may be different from the treatment site, such as a body surface site adjacent to the treatment site.

In the embodiment, the second transmission port 322 of the transmission unit 32 forms an impedance-signal detection point 322. The impedance-signal acquisition unit 33 is electrically connected with the impedance-signal detection point 322, and is configured to acquire a sampling signal from the impedance-signal detection point 322 in real time. For example, during treatment, the impedance-signal acquisition unit 33 can acquire a sampling signal from the impedance-signal detection point 322 in real time. In the embodiment, the sampling signal is a voltage signal.

The processing unit 35 is electrically connected with the impedance-signal acquisition unit 33, and configured to acquire the sampling signal and determine, according to an impedance calibration data table preset, a real-time impedance value of the living body corresponding to a real-time sampling value of the sampling signal. The impedance calibration data table pre-records a mapping relationship between multiple simulated impedance values of the living body and sampling values of multiple sampling signals.

Specifically, when the processing unit 35 acquires the sampling signal, the processing unit 35 determines the real-time sampling value of the sampling signal, and queries the real-time impedance value of the living body corresponding to the real-time sampling value of the sampling signal in the impedance calibration data table preset.

Referring to FIG. 1 again, in the embodiment, the impedance detection device 100 for the living body 200 further includes an operational amplification unit 34 electrically connected between the impedance-signal acquisition unit 33 and the processing unit 35. The operational amplification unit 34 is configured to receive the sampling signal output by the impedance-signal acquisition unit 33 and amplify the sampling signal by a preset multiple.

It can be understood that, for the sake of safety, the voltage value of the high-frequency excitation signal applied to the living body 200 is generally small, and correspondingly, the sampling value of the sampling signal acquired by the impedance-signal acquisition unit 33 is also relatively small. Therefore, a relatively big calculation error may occur to the real-time impedance value of the living body that is determined, by the processing unit 35, according to the sampling value of the sampling signal acquired by the impedance-signal acquisition unit 33. By amplifying the sampling signal via the operational amplification unit 34, the calculation accuracy of the processing unit 35 can be improved to reduce the calculation error.

The circuit structures and working principle of the above-mentioned units 31-35 will be introduced below.

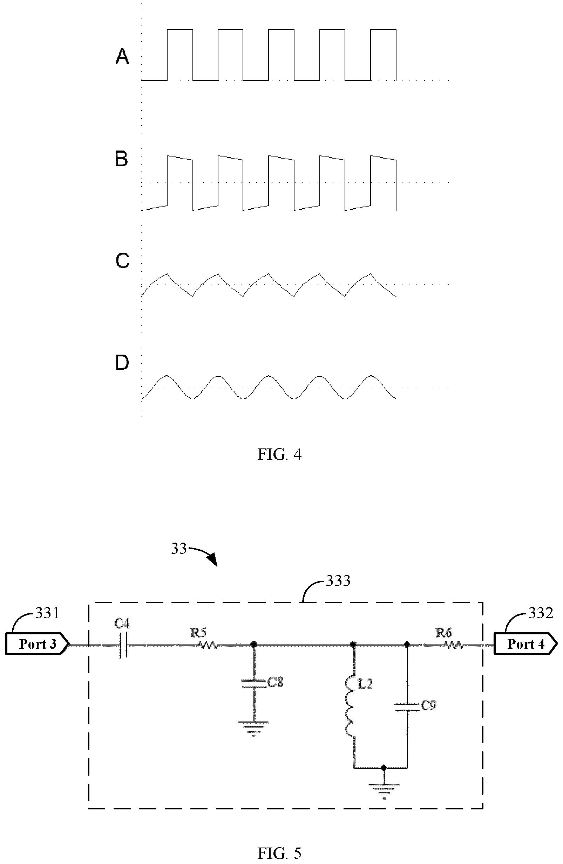

FIG. 3 is a schematic diagram of circuit structures of the excitation-signal generation unit 31 and the transmission unit 32. As illustrated in FIG. 3, the excitation-signal generation unit 31 includes a first input port 311 (Port 1), a first output port 312, and a waveform conversion circuit 313 electrically connected between the first input port 311 and the first output port 312. The first input port 311 is configured to receive an input high-frequency pulse width modulation (PWM) square-wave signal. It can be understood that the PWM square-wave signal input via the first input port 311, that is, Port 1, can be generated in various manners. For example, the processing unit 35 may be a single-chip microcomputer, and the PWM square-wave signal can be generated by the processing unit 35. For the waveform of the PWM square-wave signal, reference can be made to waveform A illustrated in FIG. 4.

In the embodiment, the waveform conversion circuit 313 is configured to convert the high-frequency PWM square-wave signal into a high-frequency sine-wave signal, that is, the high-frequency excitation signal. The waveform conversion circuit 313 includes an RC coupling circuit, an RC integrating circuit, and an LC filtering circuit electrically connected in sequence.

Specifically, the waveform conversion circuit 313 includes capacitors C1, C2, and C3, resistors R1 and R2, and an inductor L1. The first input port 311 (Port 1), the capacitor C1, the resistor R2, the inductor L1, and the first output port 312 are sequentially connected in series.

One end of the resistor R1 is electrically connected with a connection circuit between the capacitor C1 and the resistor R2, and the other end of the resistor R1 is grounded. The capacitor C1 and the resistor R1 constitute the RC coupling circuit, where the RC coupling circuit is used to filter out a direct current (DC) component in the high-frequency PWM square-wave signal, and merely an alternating current (AC) component in the high-frequency PWM square-wave signal is remained. When selecting parameters, the capacitor C1 and the resistor R1 (constituting the RC coupling circuit) need to satisfy $\tau=RC>>T$ ($\tau$ represents a time constant, R represents a resistance value, C represents a capacitance value, and T represents a cycle), and a waveform output via the RC coupling circuit is similar to a waveform input to the RC coupling circuit. When the RC coupling circuit is stable, waveform B illustrated in FIG. 4 is obtained, that is, an approximate PWM waveform is obtained by shifting the amplitude of the PWM waveform downward by 1/2.

One end of the capacitor C2 is electrically connected with a connection circuit between the resistor R2 and the inductor L1, and the other end of the capacitor C2 is grounded. The resistor R2 and the capacitor C2 constitute the RC integrating circuit that is configured to convert the high-frequency PWM square-wave signal into an approximate triangular wave or sawtooth wave signal. When selecting parameters, the resistor R2 and the capacitor C2 (constituting the RC integrating circuit) need to satisfy $\tau=RC>>T$. After the waveform B illustrated in FIG. 4 passes through the RC integrating circuit, waveform C illustrated in FIG. 4 is obtained, that is, an approximate triangle-wave waveform is obtained. The triangular wave is expanded according to the Fourier series to obtain

$$\mathrm{u}(\omega t) = \frac{8}{\pi^2} U_m (\sin \omega t - \frac{1}{9} \sin 3\omega t + \frac{1}{25} \sin 5\omega t - ...)$$

($U_m$ represents a maximum amplitude).

The capacitor C3 is connected in parallel with the inductor L1 and is electrically connected between the resistor R2 and the first output port 312. The inductor L1 and the capacitor C3 constitute a parallel frequency-selection circuit, that is, the LC filter circuit, where the LC filter circuit is configured to convert an approximate triangular wave or sawtooth wave signal into a sine-wave signal. Meanwhile, the LC filter circuit constituted by the inductor L1 and the capacitor C3 can further isolate radiofrequency current for ablation therapy. When selecting the inductor L1 and the capacitor C3 that constitute the LC filter circuit, a passband cut-off frequency of the LC filter circuit needs to be greater than the fundamental frequency of the triangle wave and less than the third harmonic frequency of the triangle wave, and the Q value (quality factor) of the inductor L1 needs to be as great as possible, to realize better frequency-selection performance. After the waveform C illustrated in FIG. 4 is filtered by the LC filter circuit, the waveform C is converted into waveform D illustrated in FIG. 4, that is, a sine-wave excitation signal that can be applied to the living body is obtained.

The first output port 312 is configured to output the sine-wave excitation signal, that is, the high-frequency excitation signal. The frequency of the high-frequency PWM square-wave signal and the frequency of the high-frequency excitation signal are the same, for example, both may be 50 kHz. There is no limitation on the frequency of the high-frequency PWM square-wave signal and the frequency of the high-frequency excitation signal in the present disclosure. In the present disclosure, coupling, integrating, and frequency-selection circuits formed by simple components constitute the excitation-signal generation unit 31 that can generate the high-frequency excitation signal of 50 kHz, and thus the excitation-signal generation unit 31 has a relatively simple circuit structure.

Referring to FIG. 3 again, the transmission unit 32 further includes a voltage-dividing resistor R3 electrically connected between the first transmission port 321 and the second transmission port 322 (Port 2), and the transmission unit 32 is configured to transmit the high-frequency excitation signal to the detection site 21 of the living body 200 via the voltage-dividing resistor R3 and the second transmission port 322.

The second transmission port 322 may be an electrode. The electrode punctures into the body tissue of the living body 200, that is, the treatment site, such as hypertrophic myocardium in the interventricular septum. After the high-frequency excitation signal is output from the first output port 312 of the excitation-signal generation unit 31, the high-frequency excitation signal is applied to the detection site 21 of the living body 200, that is, the treatment site, via the voltage-dividing resistor R3 and the electrode.

In the embodiment, the second transmission port 322 that is configured to transmit the high-frequency excitation signal and an ablation electrode that is configured to transmit a radiofrequency ablation current signal can share the same transmission channel, that is, the high-frequency excitation signal and the radiofrequency current signal for ablation can be superimposed on the ablation electrode. It can be understood that the high-frequency excitation signal is only an electrical signal applied to the living body 200 for detecting the impedance of the living body 200, and is an electrical stimulation signal of a different frequency from the radiofrequency current signal for radiofrequency ablation. In this way, the ablation electrode forms the impedance-signal detection point, and the impedance-signal acquisition unit 33 is configured to acquire the voltage of the high-frequency excitation signal at the impedance-signal detection point. It can be understood that, in another embodiment, the second transmission port 322 and the ablation electrode may correspond to different transmission channels, that is, the second transmission port 322 may be a transmission electrode independent of the ablation electrode.

FIG. 5 is a schematic diagram of a circuit structure of the impedance-signal acquisition unit 33. As illustrated in FIG. 5, the impedance-signal acquisition unit 33 includes a second input port 331 (Port 3), a second output port 332 (Port 4), and an anti-interference circuit 333 electrically connected between the second input port 331 and the second output port 332. The second input port 331 (Port 3) is electrically connected with the impedance-signal detection point 322, and the second input port 331 is configured to acquire the sampling signal from the impedance-signal detection point 322 in real time. It can be understood that, after the high-frequency excitation signal is output from the first output port 312 of the excitation-signal generation unit 31, by means of attenuation of the high-frequency excitation signal by the voltage-dividing resistor R3, a sampling signal with a reduced voltage value is obtained, and the frequency of the sampling signal is still the same as the frequency of the high-frequency excitation signal.

As mentioned above, the second transmission port 322 for transmitting the high-frequency excitation signal and the ablation electrode for transmitting the radiofrequency ablation current signal may share the same transmission channel. Therefore, there may be an interference signal in the sampling signal acquired from the impedance-signal detection point 322 in real time by the second input port 331. It can be understood that, if the second transmission port 322 and the ablation electrode do not share the same transmission channel, the radiofrequency ablation current signal can also enter the impedance-signal acquisition unit 33 from the impedance-signal detection point 322 via the living body 200, which may interfere the sampling signal. In the embodiment, the anti-interference circuit 333 is configured to process the sampling signal to filter out an interference signal in the sampling signal. The anti-interference circuit 333 includes a capacitor C4, an RC low-pass filter circuit, and an LC parallel frequency-selection circuit electrically connected in sequence.

Specifically, the anti-interference circuit 333 includes capacitors C4, C8, and C9, resistors R5 and R6, and an inductor L2. The second input port 331 (Port 3), the capacitor C4, the resistor R5, the resistor R6, and the second output port 332 (Port 4) are sequentially connected in series.

The capacitor C4 is configured to isolate the DC component in the sampling signal.

One end of the capacitor C8 is electrically connected with a connection circuit between the resistors R5 and R6, and the other end of the capacitor C8 is grounded. The resistor R5 and the capacitor C8 constitute the RC low-pass filter circuit, and the RC low-pass filter circuit is configured to allow the AC component in the sampling signal to pass through, that is, the high-frequency excitation signal, which is subjected to voltage attenuation by the transmission unit 32, mainly by the voltage-dividing resistor R3, is allowed to pass through.

The inductor L2 is connected in parallel with the capacitor C9, and is electrically connected between the connection circuit between the resistors R5 and R6 and a ground terminal. The inductor L2 and the capacitor C9 constitute the LC parallel frequency-selection circuit, and the LC parallel frequency-selection circuit is configured to filter out the interference of the power frequency in the sampling signal and the radiofrequency current signal for radiofrequency ablation aliased in the sampling signal. When selecting the inductor L2 and the capacitor C9 that constitute the LC parallel frequency-selection circuit, it needs to make the resonant frequency $f_0$ ( $f_0 = \dfrac{1}{2\pi\sqrt{LC}}$ ) of the LC parallel frequency-selection circuit equal to the frequency of the excitation signal, and to make the passband width B ( $B = \dfrac{f_0}{Q}$ ) of the LC parallel frequency-selection circuit be relatively small, that is, the Q value (quality factor) is relatively large. It can be understood that the larger the Q value, the narrower the passband of the LC parallel frequency-selection circuit and the better the filtering effect of the LC parallel frequency-selection circuit.

It can be understood that, due to relatively high energy of the radiofrequency ablation current, the interference of the radiofrequency ablation signal to the sampling signal is much greater than the interference of other signals to the sampling signal, and the radiofrequency ablation signal may cause obvious interference to the impedance detection circuit for the living body 200, and thus it needs to filter out the radiofrequency ablation signal as much as possible. In the present disclosure, by setting the anti-interference circuit 333 in the impedance-signal acquisition unit 33, a significant filtering effect can be obtained even when the radiofrequency ablation signal is aliased in the sampling signal, thereby ensuring the accuracy of impedance detection. The power frequency refers to the frequency of alternating current used in industry. The power frequency generally refers to the frequency of mains power, which is 50 Hz in China.

The impedance-signal acquisition unit 33 outputs, via the second output port 332 (Port 4) of the impedance-signal acquisition unit 33, the sampling signal processed by the anti-interference circuit 333.

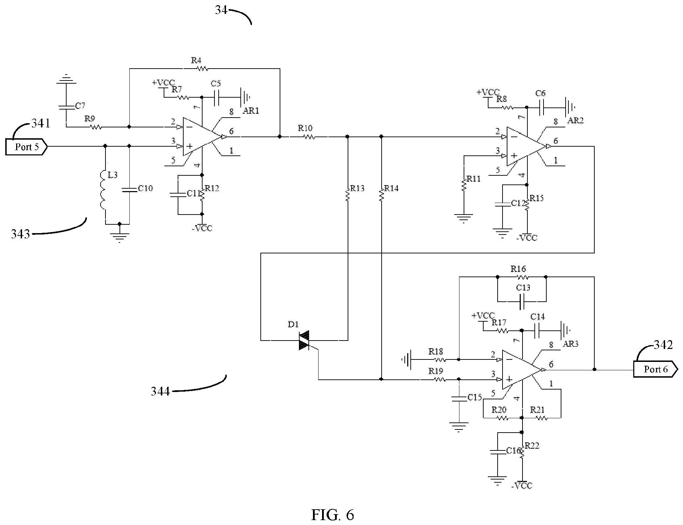

FIG. 6 is a schematic circuit diagram of the operational amplification unit 34. As illustrated in FIG. 6, the operational amplification unit 34 includes a third input port 341 (Port 5), a third output port 342 (Port 6), and a filter circuit 343 and an operational amplification circuit 344 electrically connected between the third input port 341 (Port 5) and the third output port 342 (Port 6). The third input port 341 (Port 5) is electrically connected with the second output port 332 (Port 4) of the impedance-signal acquisition unit 33, and the third input port 341 is configured to receive the sampling signal output by the impedance-signal acquisition unit 33.

The filter circuit 343 is configured to filter the sampling signal received by the third input port 341 (Port 5) to filter out the interference signal in the sampling signal, so as to ensure that there is no other interference signal in the sampling signal for operational amplification. In the embodiment, the filter circuit 343 includes an inductor L3 and a capacitor C10, the inductor L3 and the capacitor C10 are connected in parallel to constitute a parallel frequency-selection circuit, and the parallel frequency-selection circuit is electrically connected between the ground terminal and a connection circuit between the third input port 341 (Port 5) and an operational amplifier AR1.

The operational amplification circuit 344 is configured to amplify the sampling signal by a preset multiple. Specifically, in the embodiment, the operational amplification circuit 344 includes operational amplifiers AR1, AR2, AR3, and a diode D1. The operational amplifier AR1 is a non-inverting amplifier. Since the frequency of the sampling signal is relatively high, the capacitive reactance of the capacitor C7 can be ignored, so the amplification factor of the operational amplifier AR1 is $A_{u1} = 1 + \dfrac{R4}{R9}$. The operational amplifier AR2 is an inverting amplifier, and the resistance values of the resistors R13 and R14 are equal, that is, R13=R14, so the amplification factor of the operational amplifier AR2 is $A_{u2} = -\dfrac{R14}{R10}$. The diode D1 is a high-speed switch diode for rectification to obtain a DC signal for impedance calculation. The operational amplifier AR3 is a non-inverting amplifier, and the amplification factor of the operational amplifier AR3 is $A_{u3} = 1 + \dfrac{R16}{R18}$. It can be understood that the actual amplification factor of the operational amplifier circuit 344 can be adjusted according to the detection range and the detection accuracy required. Finally, the operational amplification unit 34 outputs an amplified DC voltage signal via the third output port 342 (Port 6).

It can be understood that, the sampling signal is first subjected to LC filtering pre-processing by the impedance-signal acquisition unit 33, then the sampling signal subjected to the LC filtering pre-processing is subjected to LC filtering processing before entering the operational amplification unit 34, and then after the sampling signal entering the operational amplification unit 34 is subjected to in-phase amplification, the sampling signal amplified is rectified by the diode D1 followed by secondary amplification, and thus the finally obtained sampling signal contains less interference signals, which is beneficial to improve the accuracy of detection of the impedance of the living body.

The equivalent circuit diagram of the above-mentioned impedance loop will be introduced below.

The impedance of the living body is composed of resistance, capacitive reactance, and inductive reactance. Since the inductive reactance of the living body 200 is very small, generally the inductive reactance can be ignored. The capacitive reactance of the living body 200 is sensitive to the frequency, and according to the calculation formula of the capacitive reactance $X_C = \dfrac{1}{2\pi f C}$, it can be known that the capacitive reactance is inversely proportional to the frequency, and the higher the frequency, the smaller the capacitive reactance. In the embodiment, since the excitation signal output by the excitation-signal generation unit 31 is the high-frequency excitation signal, for example, the frequency of the excitation signal is 50 kHz, the capacitive reactance at this point can also be ignored. Thus, the impedance of the living body can be approximately regarded as purely resistive.

FIG. 7 is a schematic diagram of an equivalent circuit of the impedance loop. As illustrated in FIG. 7, voltage Vo is the voltage of the high-frequency excitation signal output from the first output port 312 of the excitation-signal generation unit 31. Resistor Ro is an equivalent resistor of a transmission circuit from the first output port 312 of the excitation-signal generation unit 31 to the second transmission port 322 of the transmission unit 32, that is, the equivalent resistor of the entire transmission circuit that transmits the high-frequency excitation signal. It can be understood that the resistance value of the equivalent resistor Ro includes resistance values of the voltage-dividing resistor R3, transmission cables and so on. In the embodiment, the resistance value of the voltage-dividing resistor R3 is relatively large. Resistor Rx is an equivalent impedance of the living body to be measured. Since the living body 200 is connected with the neutral electrode 41, the resistor Rx can be equivalent to be grounded. Voltage Vadc is a voltage at the impedance-signal detection point 322. That is, in the embodiment, the sampling signal is the voltage Vadc.

According to the principle of resistor divider, it can be known that $\dfrac{Uo}{Ro + Rx} = \dfrac{Uadc}{Rx}$, where Uo is the voltage value of the voltage Vo, and Uadc is the voltage value of the voltage Vadc. In this way, the relationship between the impedance Rx of the living body and the voltage value Uadc of the sampling signal Vadc can be obtained as:

$$Rx = \frac{Uadc * Ro}{Uo - Uadc}.$$

FIG. 8 is a schematic diagram illustrating a curve relationship between the voltage value Uadc of the sampling signal Vadc and the impedance value of the impedance Rx of the living body. It can be seen from FIG. 8 that the voltage value Uadc of the sampling signal Vadc is positively correlated with the impedance Rx of the living body, and the relationship is approximately linear within a certain range.

Referring to FIG. 7 again, in the embodiment, the processing unit 35 includes a data acquisition module 351 and a calculation module 352. The data acquisition module 351 can be an ADC acquisition module for acquiring the sampling signal Vadc and determining the real-time sampling value of the sampling signal Vadc. It can be understood that, in the embodiment, the sampling signal Vadc acquired by the data acquisition module 351 is a sampling signal processed and amplified by the impedance-signal acquisition unit 33 and the operational amplification unit 34.

In the embodiment, the calculation module 352 is configured to determine the real-time impedance value of the living body corresponding to the real-time sampling value of the sampling signal according to the impedance calibration data table preset.

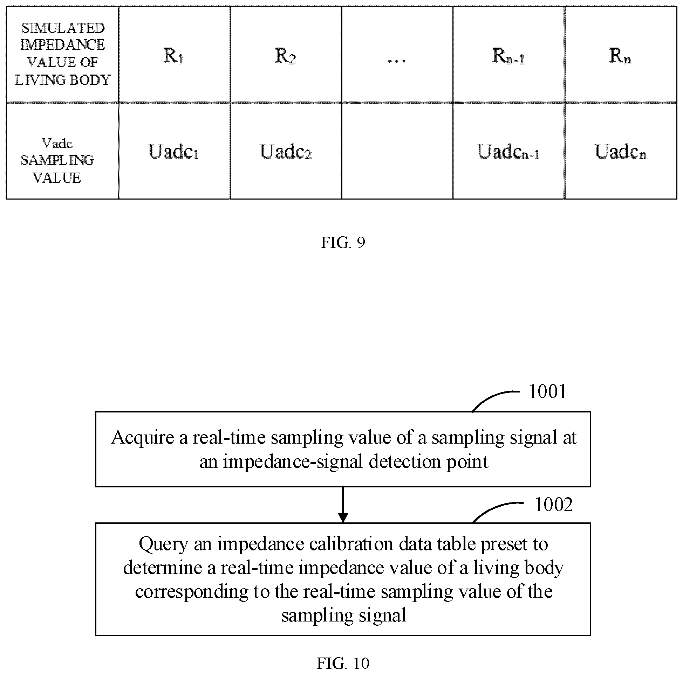

FIG. 9 is a schematic diagram of the impedance calibration data table preset. The impedance detection device 100 for the living body 200 may further include a memory 42, and the impedance calibration data table may be pre-stored in the memory 42. As illustrated in FIG. 9, the impedance calibration data table pre-records the mapping relationship between multiple simulated impedance values of the living body and sampling values of multiple sampling signals.

The impedance calibration data table can be prepared in advance before the device leaves the factory, and the data recorded in the impedance calibration data table can be determined in the following manner: using multiple high-precision non-inductive resistors to simulate the impedance of the living body 200, using the above-mentioned impedance detection device 100 to detect the sampling signal at the impedance-signal detection point, recording voltage values of sampling signals acquired by the data acquisition module 351 under different simulated impedance values of the living body (that is, the voltage value of the voltage signal transmitted to the data acquisition module 351 after the voltage signal at the impedance-signal detection point passes through the impedance-signal acquisition unit 33 and the operational amplification unit 34), and finally preparing the impedance calibration data table according to the correspondence between multiple simulated impedance values of the living body and voltage values of multiple sampling signals.

In the embodiment, the calculation module 352 is configured to invoke the impedance calibration data table preset and query in the impedance calibration data table the real-time impedance value of the living body corresponding to the real-time sampling value of the sampling signal.

Specifically, if the real-time sampling value of the sampling signal Vadc acquired by the data acquisition module 351, that is, the voltage value $Uadc_x$, is equal to a certain sampling value $Uadc_n$ in the impedance calibration data table, the simulated impedance value $R_n$ of the living body corresponding to the sampling value $Uadc_n$ in the impedance calibration data table is the real-time impedance value of the living body 200.

In the embodiment, the calculation module 352 is further configured to query, in the impedance calibration data table, two sampling values proximate to the real-time sampling value of the sampling signal and simulated impedance values of the living body respectively corresponding to the two sampling values on condition that the real-time impedance value of the living body corresponding to the real-time sampling value of the sampling signal is not found in the impedance calibration data table, and calculate the real-time impedance value of the living body corresponding to the real-time sampling value of the sampling signal according to the simulated impedance values of the living body respectively corresponding to the two sampling values and a preset linear formula.

Specifically, as mentioned above, the voltage value Uadc of the sampling signal Vadc is positively correlated with the impedance Rx of the living body, and has an approximately linear relationship within a certain range. Assuming that the real-time sampling value of the current sampling signal is Uadc $_x$, the two sampling values proximate to the real-time sampling value of the sampling signal in the impedance calibration data table are Uadc $_{n-1}$ and Uadc $_n$, that is, Uadc $_{n-1}$ < Uadc $_x$ < Uadc $_n$, and the simulated impedance values of the living body respectively corresponding to the two sampling values Uadc $_{n-1}$ and Uadc $_n$ are R $_{n-1}$ and $R_n$, respectively, according to the straight-line two-point equation

$$\frac{Uadc_x - Uadc_{n-1}}{Uadc_n - Uadc_{n-1}} = \frac{R_x - R_{n-1}}{R_n - R_{n-1}}$$

, it can be known that the real-time impedance value Rx of the living body can be calculated according to the following linear formula:

$$R_x = \frac{(R_n - R_{n-1}) * (Uadc_x - Uadc_{n-1})}{Uadc_n - Uadc_{n-1}} + R_{n-1} .$$

It can be understood that the more data recorded in the impedance calibration data table, the more beneficial it is to improve the calculation accuracy of the real-time impedance value of the living body.

The impedance detection device 100 of the present disclosure determines the real-time impedance value of the living body 200 according to the characteristics that impedance values of the living body 200 and sampling values of sampling signals have an approximately linear relationship within a certain range and the impedance calibration data table preset, and thus the impedance detection device 100 can respond quickly and can accurately and quickly detect the real-time impedance value of the living body 200, so that the change of the impedance of the living body can be monitored in real time during treatment, thereby providing reference information for medical staff to ensure safe operation of treatment. Specifically, the manner in which the real-time impedance value of the living body is determined includes determining the real-time impedance value of the living body through directly looking up the table during detection, or determining the real-time impedance value of the living body through looking up the table in sections and formula calculation.

It can be understood that since the resistance value of the equivalent resistor Ro includes the resistance values of transmission cables and the like in addition to the resistance value of the voltage-dividing resistor R3, the resistance value of the equivalent resistance Ro actually measured may have a certain error. If the real-time impedance value Rx of the living body is calculated according to the above-mentioned voltage-dividing formula $Rx = \dfrac{Uadc * Ro}{Uo - Uadc}$ , errors may occur to the real-time impedance value Rx of the living body calculated.

The impedance detection device 100 of the present disclosure determines the real-time impedance value of the living body 200 through presetting the impedance calibration data table, and through looking up the table during detection or through looking up the table in sections and formula calculation, and thus the calculation error can be effectively eliminated, thereby improving the precision of detection of the impedance of the living body 200, and preventing the error that occurs to measurement of the actual resistance of the equivalent resistor Ro from affecting the calculation of the real-time impedance value Rx of the living body.

In addition, the impedance detection device 100 of the present disclosure can detect the impedance value of the living body 200 through generating an excitation signal with a fixed voltage, only voltage sampling is performed, and current sampling is not performed, and thus the detection circuit is simple, thereby reducing the cost and volume of the impedance detection device 100 for the living body 200, and meanwhile, the impedance detection device 100 for the living body 200 can have a relatively high dynamic response speed. In addition, since the impedance detection device 100 for the living body 200 has a relatively simple circuit structure, the impedance loop of the living body can be equivalent to a simple resistance voltage-dividing model, which results in that the impedance value of the living body 200 actually detected is more accurate and reliable.

The present disclosure further provides an impedance detection method for a living body, which is realized by the above-mentioned impedance detection device 100 for the living body 200. FIG. 10 is a flow chart of the impedance detection method for the living body 200 provided by embodiments of the present disclosure. It is to be noted that the impedance detection method for the living body 200 in the embodiments of the present disclosure is not limited to the steps and sequence in the flow chart illustrated in FIG. 10. According to different requirements, additional steps can be added to the flow chart illustrated, some steps in the flow chart illustrated can be removed, or the sequence of the steps in the flow chart illustrated can be changed. As illustrated in FIG. 10, the impedance detection method for the living body includes the following steps.

Step 1001, acquire a real-time sampling value of a sampling signal at an impedance-signal detection point.

For technical details of the sampling signal at the impedance-signal detection point, reference can be made to specific illustration of the impedance detection device 100 for the living body 200, which will not be repeated herein.

Step 1002, query an impedance calibration data table preset to determine a real-time impedance value of the living body corresponding to the real-time sampling value of the sampling signal.

The impedance calibration data table pre-records a mapping relationship between multiple simulated impedance values of the living body and sampling values of multiple sampling signals.

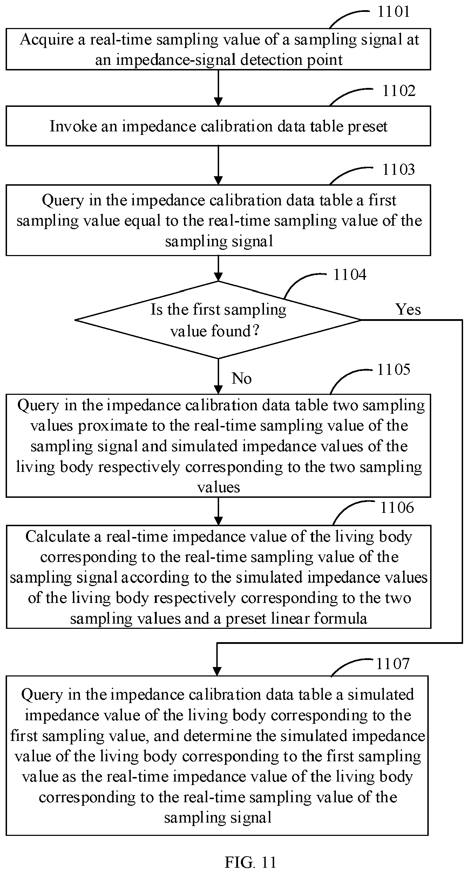

FIG. 11 is a flow chart of another impedance detection method for a living body provided by embodiments of the present disclosure. As illustrated in FIG. 11, the impedance detection method for the living body includes the following steps.

Step 1101, acquire a real-time sampling value of a sampling signal at an impedance-signal detection point.

Step 1102, invoke an impedance calibration data table preset.

The impedance calibration data table pre-records a mapping relationship between multiple simulated impedance values of the living body and sampling values of multiple sampling signals.

Step 1103, query in the impedance calibration data table a first sampling value equal to the real-time sampling value of the sampling signal.

Step 1104, determine whether the first sampling value is found in the impedance calibration data table. If the first sampling value is not found, step 1105 is performed. If the first sampling value is found, step 1107 is performed.

Step 1105, query in the impedance calibration data table two sampling values proximate to the real-time sampling value of the sampling signal and simulated impedance values of the living body respectively corresponding to the two sampling values.

Step 1106, calculate a real-time impedance value of the living body corresponding to the real-time sampling value of the sampling signal according to the simulated impedance values of the living body respectively corresponding to the two sampling values and a preset linear formula.

As stated above, the preset linear formula can be set to be
$$R_x = \frac{(R_n - R_{n-1}) * (Uadc_x - Uadc_{n-1})}{Uadc_n - Uadc_{n-1}} + R_{n-1}$$
, where $Uadc_x$ is the real-time sampling value of the sampling signal, $Uadc_{n-1}$ and $Uadc_n$ are two sampling values proximate to the real-time sampling value $Uadc_x$ of the sampling signal in the impedance calibration data table, that is, $Uadc_{n-1} < Uadc_x < Uadc_n$, $R_{n-1}$ and $R_n$ are simulated impedance values of the living body respectively corresponding to the two sampling values $Uadc_{n-1}$ and $Uadc_n$.

Step 1107, query in the impedance calibration data table a simulated impedance value of the living body corresponding to the first sampling value, and determine the simulated impedance value of the living body corresponding to the first sampling value as the real-time impedance value of the living body corresponding to the real-time sampling value of the sampling signal.

In the impedance detection method for the living body of the present disclosure, according to the characteristics that impedance values of the living body and sampling values of sampling signals have an approximately linear relationship within a certain range and the impedance calibration data table preset, the real-time impedance value of the living body can be determined through looking up the table during detection or through looking up the table in sections and formula calculation during detection, and thus the real-time impedance value of the living body can be accurately and quickly detected, so that the change of the impedance of the living body can be monitored in real time during treatment, thereby providing reference information for medical staff to ensure safe operation of treatment.

Those skilled in the art can understand that the aforementioned schematic diagram 1 illustrates only an example of the impedance detection device 100 for the living body 200 used to implement the impedance detection method for the living body 200 in the present disclosure, and does not constitute a limitation to the impedance detection device 100 for the living body 200. The impedance detection device 100 for the living body 200 may include more or fewer components than the figures illustrated, or include different components from the figures illustrated, or certain components in the figures illustrated can be combined, for example, the impedance detection device 100 for the living body 200 may further include a display unit and the like.

The memory 42 may include a high-speed random access memory, may also include a non-volatile memory, such as a hard disk, an internal memory, a plug-in hard disk, a smart memory card (SMC), a secure digital (SD) card, a flash card, at least one magnetic disk storage device, a flash memory, or other volatile solid-state storage devices.

A computer program may also be stored in the memory 42. The computer program can be divided into one or more modules/units, and the one or more modules/units are stored in the memory 42 and executed by the processing unit 35 to complete the impedance detection method for the living body in the present disclosure. The one or more modules/units may be a series of computer program instruction segments capable of accomplishing specific functions, and the instruction segments are used to describe the execution process of the computer program in the impedance detection device 100 for the living body 200.

The processing unit 35 may be a single-chip microcomputer, a central processing unit (CPU), and may also be other general-purpose processors, a digital signal processor (DSP), an application specific integrated circuit (ASIC), a field-programmable gate array (FPGA) or other programmable logic devices, a discrete gate or transistor logic device, a discrete hardware component, etc. The processing unit 35 realizes detection of the impedance of the living body by running or executing the computer program and/or modules/units stored in the memory 42 and invoking data stored in the memory 42.

In the embodiment, the processing unit 35 executes the computer program to realize the steps in each impedance detection method for the living body mentioned above, such as steps 1001-1002 illustrated in FIG. 10, or steps 1101~1106 illustrated in FIG. 11.

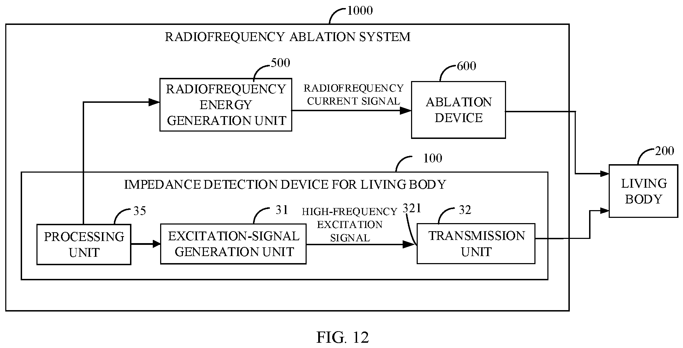

The present disclosure further provides a radiofrequency ablation system using the above-mentioned impedance detection device 100 for the living body 200. FIG. 12 is a schematic structural diagram of the radiofrequency ablation system. As illustrated in FIG. 12, the radiofrequency ablation system 1000 includes a radiofrequency energy generation unit 500, an ablation device 600, and the above-mentioned impedance detection device 100. The radiofrequency energy generation unit 500 is configured to generate a radiofrequency current signal with a set power during radiofrequency ablation, so as to provide radiofrequency energy required for radiofrequency ablation. The impedance detection device 100 for the living body 200 is configured to detect an impedance value of the living body 200 in real time during radiofrequency ablation to monitor the change of the impedance value of the living body 200, thereby assisting safe operation of radiofrequency ablation.

The excitation-signal generation unit 31 and the radiofrequency energy generation unit 500 of the impedance detection device 100 for the living body 200 may be two independent devices, or may be disposed in the same device.

The radiofrequency energy generation unit 500 is further electrically connected with the ablation device 600 (such as an ablation electrode). The ablation device 600 is configured to be inserted into an ablation site (such as the above-mentioned treatment site of the living body 200) during radiofrequency ablation, receive radiofrequency energy output by the radiofrequency energy generation unit 500, and release the radiofrequency energy to the ablation site to perform radiofrequency ablation on the ablation site, so as to treat lesion tissue. The ablation site/treatment site refers to a lesion site of the living body 200, such as lesion tissue of the heart or other lesion tissue. Taking hypertrophic cardiomyopathy as an example, the ablation device 600 is inserted into the patient's heart through transapical approach to perform radiofrequency ablation on hypertrophic interventricular septal myocardium to treat hypertrophic cardiomyopathy.

In the embodiment, the detection site 21 and the treatment site are the same site. In this way, as mentioned above, in the impedance detection device 100 for the living body 200, the second transmission port 322 for transmitting the high-frequency excitation signal and the ablation electrode for transmitting the radiofrequency ablation current signal can share the same transmission channel, That is to say, the transmission unit 32 may be partially or fully disposed in the ablation device 600.

In the embodiment, the processing unit 35 of the impedance detection device 100 for the living body 200 is further electrically connected with the excitation-signal generation unit 31 and the radiofrequency energy generation unit 500, and is configured to provide a high-frequency PWM square-wave signal for the excitation-signal generation unit 31, analyze the change of the impedance value of the living body within a preset time range according to the impedance value of the living body 200 actually determined, and adjust the radiofrequency current signal output by the radiofrequency energy generation unit 500 according to the analysis result, such as adjusting the output state and/or size of the radiofrequency current signal, thereby avoiding abnormalities such as carbonization, scabbing or shedding of cell tissue of the living body 200 at the ablation site during radiofrequency ablation, which in turn avoids expansion of the wounded area, massive bleeding, and even perforation at the treatment site.

It can be seen that by using the above-mentioned impedance detection device 100 for the living body 200 in the radiofrequency ablation system 1000, an effective solution can be provided for real-time detection of the impedance of the living body during radiofrequency ablation.

Finally, it is to be noted that the above embodiments are only used to illustrate the technical solutions of the present disclosure rather than limiting the present disclosure. Although the present disclosure has been described in detail with reference to the above preferred embodiments, those of ordinary skill in the art should understand that modification or equivalent replacement of the technical solutions of the present disclosure do not deviate from the spirit and scope of the technical solutions of the present disclosure.

**Claims**

1. An impedance detection device for a living body, comprising:

   an excitation-signal generation unit configured to generate and output a high-frequency excitation signal;
   a transmission unit comprising a first transmission port and a second transmission port, wherein the transmission unit is electrically connected with the excitation-signal generation unit via the first transmission port to receive the high-frequency excitation signal, and is configured to transmit the high-frequency excitation signal to a detection site of the living body via the second transmission port, and wherein the second transmission port forms an impedance-signal detection point;

an impedance-signal acquisition unit electrically connected with the impedance-signal detection point, and configured to acquire a sampling signal from the impedance-signal detection point in real time; and

a processing unit electrically connected with the impedance-signal acquisition unit, and configured to acquire the sampling signal and determine a real-time impedance value of the living body corresponding to a real-time sampling value of the sampling signal according to an impedance calibration data table preset, wherein the impedance calibration data table pre-records a mapping relationship between a plurality of simulated impedance values of the living body and sampling values of a plurality of sampling signals.

2. The impedance detection device for the living body of claim 1, wherein the processing unit comprises a data acquisition module and a calculation module, wherein the data acquisition module is configured to acquire the sampling signal and determine the real-time sampling value of the sampling signal; and

the calculation module is configured to invoke the impedance calibration data table preset and query in the impedance calibration data table the real-time impedance value of the living body corresponding to the real-time sampling value of the sampling signal.

3. The impedance detection device for the living body of claim 2, wherein the calculation module is further configure to query, in the impedance calibration data table, two sampling values proximate to the real-time sampling value of the sampling signal and simulated impedance values of the living body respectively corresponding to the two sampling values on condition that the real-time impedance value of the living body corresponding to the real-time sampling value of the sampling signal is not found in the impedance calibration data table, and to calculate the real-time impedance value of the living body corresponding to the real-time sampling value of the sampling signal according to the simulated impedance values of the living body respectively corresponding to the two sampling values and a preset linear formula.

4. The impedance detection device for the living body of claim 1, wherein the transmission unit further comprises a voltage-dividing resistor electrically connected between the first transmission port and the second transmission port, and is configured to transmit the high-frequency excitation signal to the detection site of the living body via the voltage-dividing resistor and the second transmission port.

5. The impedance detection device for the living body of any one of claims 1 to 4, further comprising an operational amplification unit electrically connected between the impedance-signal acquisition unit and the processing unit, wherein the operational amplification unit is configured to receive the sampling signal output by the impedance-signal acquisition unit and amplify the sampling signal by a preset multiple.

6. The impedance detection device for the living body of claim 5, wherein the operational amplification unit comprises a filter circuit and an operational amplification circuit, wherein the filter circuit is configured to filter the sampling signal received by the operational amplification unit to filter out an interference signal in the sampling signal, and the operational amplification circuit is configured to amplify the sampling signal by the preset multiple.

7. The impedance detection device for the living body of any one of claims 1 to 4, wherein the excitation-signal generation unit comprises a first input port, a first output port, and a waveform conversion circuit electrically connected between the first input port and the first output port, wherein

the first input port is configured to receive a high-frequency pulse width modulation (PWM) square-wave signal;
the waveform conversion circuit is configured to convert the high-frequency PWM square-wave signal into a high-frequency sine-wave signal, wherein the high-frequency sine-wave signal is the high-frequency excitation signal; and
the first output port is configured to output the high-frequency excitation signal.

8. The impedance detection device for the living body of claim 7, wherein the processing unit is further electrically connected with the excitation-signal generation unit, and configured to provide the high-frequency PWM square-wave signal for the excitation-signal generation unit.

9. The impedance detection device for the living body of any one of claims 1 to 4, wherein the impedance-signal acquisition unit further comprises an anti-interference circuit, wherein the anti-interference circuit is configured to process the sampling signal to filter out an interference signal in the sampling signal.

10. The impedance detection device for the living body of claim 9, wherein the anti-interference circuit comprises a capacitor, an RC low-pass filter circuit, and an LC parallel frequency-selection circuit electrically connected in sequence, wherein

the capacitor is configured to isolate a direct current (DC) component in the sampling signal; the RL low-pass filter circuit is configured to allow an alternating current (AC) component in the sampling signal to pass through; and
the LC parallel frequency-selection circuit is configured to filter out interference of a power frequency in the sampling signal and a radiofrequency current signal for radiofrequency ablation aliased in the sampling signal.

11. A radiofrequency ablation system comprising a radiofrequency energy generation unit, an ablation device, and the impedance detection device for the living body of any one of claims 1 to 10, wherein the radiofrequency energy generation unit is configured to provide radiofrequency energy for radiofrequency ablation; and
the ablation device is electrically connected with the radiofrequency energy generation unit, is configured to be inserted into a treatment site of the living body during radiofrequency ablation, to receive the radiofrequency energy output by the radiofrequency energy generation unit, and to release the radiofrequency energy to the treatment site to perform radiofrequency ablation on the treatment site.

12. The radiofrequency ablation system of claim 11, wherein the detection site and the treatment site are the same site, and the transmission unit is partially or fully disposed in the ablation device.

13. The radiofrequency ablation system of claim 11, wherein the processing unit of the impedance detection device for the living body is further electrically connected with the radiofrequency energy generation unit, and the processing unit is configured to analyze a change of an impedance value of the living body within a preset time range according to an impedance value of the living body actually determined, and to adjust a radiofrequency current signal output by the radiofrequency energy generation unit according to the analysis result.

┌─────────────────────────────────────────────────────────────────────────┐
│                    ⌐ 100                                                   │
│  IMPEDANCE DETECTION DEVICE FOR  LIVING BODY                              │
│                                                                           │
│         ⌐ 31        HIGH-FREQUENCY    321    ⌐ 32                          │
│  ┌──────────────┐   EXCITATION SIGNAL    ┌────────────┐                    │
│  │ EXCITATION-  │ ──────────────────────▶│TRANSMISSION│                    │
│  │   SIGNAL     │                        │    UNIT    │                    │
│  │ GENERATION   │                        └────────────┘                    │
│  │    UNIT      │                                                          │
│  └──────────────┘                                                          │
└─────────────────────────────────────────────────────────────────────────┘

FIG. 1

IMPEDANCE DETECTION DEVICE FOR  LIVING BODY — 100

EXCITATION-SIGNAL GENERATION UNIT — 31

HIGH-FREQUENCY EXCITATION SIGNAL — 321

TRANSMISSION UNIT — 32

322

LIVING BODY — 200

IMPEDANCE-SIGNAL ACQUISITION UNIT — 33

OPERATIONAL AMPLIFICATION UNIT — 34

MEMORY — 42

PROCESSING UNIT — 35

FIG. 1

FIG. 2

FIG. 3

FIG. 4

FIG. 5

FIG. 6

Vo

35

PROCESSING UNIT

42

MEMORY

352

CALCULATION MODULE

351

DATA ACQUISITION MODULE

Vadc

Ro

322

Rx

FIG. 7

dc

$dc_n$

$dc_x$

$c_{n-1}$

O

$R_{n-1}$ $R_x$ $R_n$

FIG. 8

| SIMULATED IMPEDANCE VALUE OF LIVING BODY | $R_1$ | $R_2$ | ... | $R_{n-1}$ | $R_n$ |
|---|---|---|---|---|---|
| Vadc SAMPLING VALUE | $Uadc_1$ | $Uadc_2$ | | $Uadc_{n-1}$ | $Uadc_n$ |

FIG. 9

1001

Acquire a real-time sampling value of a sampling signal at an impedance-signal detection point

1002

Query an impedance calibration data table preset to determine a real-time impedance value of a living body corresponding to the real-time sampling value of the sampling signal

FIG. 10

1101

Acquire a real-time sampling value of a sampling signal at an impedance-signal detection point

1102

Invoke an impedance calibration data table preset

1103

Query in the impedance calibration data table a first sampling value equal to the real-time sampling value of the sampling signal

1104

Is the first sampling value found?

Yes

No

1105

Query in the impedance calibration data table two sampling values proximate to the real-time sampling value of the sampling signal and simulated impedance values of the living body respectively corresponding to the two sampling values

1106

Calculate a real-time impedance value of the living body corresponding to the real-time sampling value of the sampling signal according to the simulated impedance values of the living body respectively corresponding to the two sampling values and a preset linear formula

1107

Query in the impedance calibration data table a simulated impedance value of the living body corresponding to the first sampling value, and determine the simulated impedance value of the living body corresponding to the first sampling value as the real-time impedance value of the living body corresponding to the real-time sampling value of the sampling signal

FIG. 11

FIG. 12

## INTERNATIONAL SEARCH REPORT

| International application No. |
| --- |
| **PCT/CN2021/083077** |

**A. CLASSIFICATION OF SUBJECT MATTER**

A61B 18/12(2006.01)i; A61B 18/14(2006.01)i; A61B 5/053(2021.01)i; A61B 90/00(2016.01)i

According to International Patent Classification (IPC) or to both national classification and IPC

**B. FIELDS SEARCHED**

Minimum documentation searched (classification system followed by classification symbols)

A61B18, A61B5, A61B90

Documentation searched other than minimum documentation to the extent that such documents are included in the fields searched

Electronic data base consulted during the international search (name of data base and, where practicable, search terms used)

CNABS, VEN.CNKI: 杭州诺诚, 刘道洋, 王雄志, 胡普锋, 丘信炯, 阻抗, 激励, 消融, 表格electrode?, impedance?, ablat+, impedant+, data, table, sheet

**C. DOCUMENTS CONSIDERED TO BE RELEVANT**

| Category* | Citation of document, with indication, where appropriate, of the relevant passages | Relevant to claim No. |
| --- | --- | --- |
| X | CN 104582619 A (MEDTRONIC ABLATION FRONTIERS, LLC.) 29 April 2015 (2015-04-29) <br> description, paragraphs [0022]-[0041] and figures 1-7 | 1-13 |
| X | US 2018325456 A1 (BOSTON SCIENT SCIMED INC.) 15 November 2018 (2018-11-15) <br> description, paragraphs [0048]-[0063] and figures 1-5 | 1-13 |
| X | US 2017143414 A1 (ST JUDE MEDICAL CARDIOLOGY DIV INC.) 25 May 2017 (2017-05-25) <br> description, paragraphs [0032]-[0045] and figure 1 | 1-13 |
| X | CN 103379873 A (MEDTRONIC ABLATION FRONTIERS, LLC.) 30 October 2013 (2013-10-30) <br> description, paragraphs [0027]-[0053] and figure 1 | 1-13 |
| X | CN 111491560 A (HOCHSCHULE OFFENBURG) 04 August 2020 (2020-08-04) <br> abstract | 1-13 |
| X | CN 204428153 U (ZHUHAI HOKAI MEDICAL INSTRUMENTS CO., LTD.) 01 July 2015 (2015-07-01) <br> description, paragraphs [0017]-[0023] and figure 1 | 1-13 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **23 May 2021** | **28 May 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/083077** |

**C.    DOCUMENTS CONSIDERED TO BE RELEVANT**

| Category* | Citation of document, with indication, where appropriate, of the relevant passages | Relevant to claim No. |
| --- | --- | --- |
| X | CN 111214288 A (HANGZHOU DINOVA MEDICAL DEVICES CO., LTD.) 02 June 2020 (2020-06-02) <br> description, paragraphs [0043]-[0206] and figures 1-7 | 1-13 |
| A | US 2011098695 A1 (VIVANT MEDICAL INC.) 28 April 2011 (2011-04-28) <br> entire document | 1-13 |

Form PCT/ISA/210 (second sheet) (January 2015)

## INTERNATIONAL SEARCH REPORT
### Information on patent family members

International application No.

**PCT/CN2021/083077**

| Patent document cited in search report | | | Publication date (day/month/year) | Patent family member(s) | | | Publication date (day/month/year) |
|---|---|---|---|---|---|---|---|
| CN | 104582619 | A | 29 April 2015 | EP | 2840996 | A1 | 04 March 2015 |
| | | | | EP | 2840996 | B1 | 20 March 2019 |
| | | | | CN | 104582619 | B | 04 December 2018 |
| | | | | WO | 2013162884 | A1 | 31 October 2013 |
| US | 2018325456 | A1 | 15 November 2018 | WO | 2018208951 | A1 | 15 November 2018 |
| US | 2017143414 | A1 | 25 May 2017 | WO | 2017087740 | A1 | 26 May 2017 |
| | | | | EP | 3359073 | B1 | 12 February 2020 |
| | | | | EP | 3359073 | A1 | 15 August 2018 |
| CN | 103379873 | A | 30 October 2013 | US | 9265557 | B2 | 23 February 2016 |
| | | | | US | 10166071 | B2 | 01 January 2019 |
| | | | | WO | 2012106100 | A2 | 09 August 2012 |
| | | | | US | 2016128603 | A1 | 12 May 2016 |
| | | | | US | 2012197243 | A1 | 02 August 2012 |
| | | | | CN | 103379873 | B | 31 August 2016 |
| | | | | EP | 2670329 | A2 | 11 December 2013 |
| CN | 111491560 | A | 04 August 2020 | EP | 3706626 | A1 | 16 September 2020 |
| | | | | WO | 2019091991 | A1 | 16 May 2019 |
| | | | | US | 2020261024 | A1 | 20 August 2020 |
| CN | 204428153 | U | 01 July 2015 | None | | | |
| CN | 111214288 | A | 02 June 2020 | None | | | |
| US | 2011098695 | A1 | 28 April 2011 | EP | 2316371 | A1 | 04 May 2011 |
| | | | | US | 8568401 | B2 | 29 October 2013 |
| | | | | JP | 2018047350 | A | 29 March 2018 |
| | | | | US | 2018296266 | A1 | 18 October 2018 |
| | | | | JP | 6272407 | B2 | 31 January 2018 |
| | | | | US | 2015126991 | A1 | 07 May 2015 |
| | | | | EP | 2316371 | B1 | 05 March 2014 |
| | | | | US | 8894641 | B2 | 25 November 2014 |
| | | | | JP | 2015006370 | A | 15 January 2015 |
| | | | | JP | 2011092715 | A | 12 May 2011 |
| | | | | JP | 5580168 | B2 | 27 August 2014 |
| | | | | US | 2014052124 | A1 | 20 February 2014 |
| | | | | US | 10004559 | B2 | 26 June 2018 |
| | | | | JP | 2016179386 | A | 13 October 2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010918869X

- CN 202021909126